# EUROPEAN PATENT SPECIFICATION

(11) **EP 1 906 921 B1**
(45) Date of publication and mention of the grant of the patent: **03.12.2008**
(21) Application number: 05771847.0
(22) Date of filing: 08.07.2005
(51) Int. Cl.: A61K 9/107, A61K 31/05, A61K 47/12

(54) **CLEAR PHARMACEUTICAL AQUEOUS MICROEMULSION COMPRISING PROPOFOL AND PROCESS FOR PREPARATION**
KLARE PHARMAZEUTISCHE WÄSSRIGE MIKROEMULSION MIT PROPOFOL UND HERSTELLUNGSVERFAHREN
MICROEMULSION PHARMACEUTIQUE AQUEUSE ET TRANSPARENTE COMPRENANT DU PROPOFOL, ET SON PROCEDE DE PREPARATION

(43) Date of publication of application: 09.04.2008
(73) Proprietor: Physica Pharma, 33600 Pessac (FR)
(72) Inventor: POUGNAS, Jean-Luc, F-33500 Libourne (FR); BROUSSAUD, Olivier, F-33000 Bordeaux (FR); CALVET, Nicolas, F-33200 Bordeaux (FR); COMTAT, Sophie, F-33000 Bordeaux (FR)
(74) Representative: Bolinches, Michel Jean-Marie
(86) International application number: PCT/EP2005/008739
(87) International publication number: WO 2007/006334

(56) References cited:
- WO-A-00/78301
- WO-A-02/09671
- WO-A-03/015823
- WO-A-20/04032910
- US-A- 4 798 846
- US-A1- 2003 138 489

## Description

The present invention concerns a clear pharmaceutical aqueous emulsion composition which comprises Propofol and which allows a safe administration, particularly through intravenous injection. The invention also concerns a process for preparing this composition.

Propofol (i.e. 2,6-diisopropylphenol) is a well-known intravenous anesthetic agent which has been used in pharmaceutical aqueous emulsion compositions since the early 1980's. Because of the poor solubility of Propofol in water resulting from its lipophilic nature, intravenous formulations containing Propofol needed to be optimised in order to keep the drug dissolved in a safe composition.

The first Propofol composition which appeared on the market was an intravenous opaque and stable macro-emulsion named "Intralipid". This composition contained triglycerides such as soybean oil, which were included at a mass fraction typically of about 10%, see e.g. the "Diprivan" compositions from Astra Zeneca Pharmaceuticals which also essentially comprise a phospholipid and glycerin.

One major drawback of such triglycerides-containing emulsions resides in their unclear nature and in the relatively large medium particle size in the emulsions - typically greater than 150 nm (nanometers) - which may lead to the formation of clots in blood capillaries and other vessels, thus rendering these triglycerides containing compositions not totally safe through intravenous injection.

In order to minimize potential side effects due to high triglycerides content, some searchers have focused their works in developing triglycerides-free formulations with the aim to obtain clear aqueous compositions, as detailed below.

Patent document US-B-6,623,765 discloses in its examples a clear aqueous micro-emulsion composition comprising 1% (v/v) Propofol and at least 4.05% (w/v) of a surfactant system including at least 0.05% (w/v) of sodium laurate and at least 4% of a poloxamer (i.e. a block copolymer of ethylene oxide / propylene oxide).

Patent document WO-A-00/78301 discloses in its examples another clear aqueous micro-emulsion composition essentially comprising 1 g of Propofol and at least 5.5 g of a surfactant system including one or several poloxamer(s) and a polyethylene glycol hydroxystearate.

One major disadvantage of the compositions that were tested in these documents resides in their relatively high ratio surfactant system /Propofol, which is taught to be greater than or equal to 4.05% (w/w) in US-B-6,623,765 and to 5.5% in WO-A-00/78301 which results in a risk of potential side effects after intravenous injection of these compositions.

One purpose of the present invention is to overcome this disadvantage, and this is achieved in that the Applicant has surprisingly discovered that the combination, in a surfactant system for a pharmaceutical aqueous emulsion composition comprising Propofol, of:
- at least one pharmaceutically acceptable monovalent metal salt of a fatty acid having from 5 to 23 carbon atoms, and of
- at least one polyethylene glycol hydroxystearate,
permits to obtain a clear composition at a ratio surfactant system / Propofol lower than 4, which was totally unexpected in the prior art with such a reduced ratio, thus minimizing any alteration phenomenon of physiological parameters such as haemolysis or risk of intolerance, after intravenous injection of this composition.

It is to be noted here that the present invention resides in the unexpected and advantageous synergistic effect of said combination of monovalent metal salt(s) of fatty acid and polyethylene glycol hydroxystearate(s), in comparison to the state of the art disclosing either such a metal salt alone (see above-mentioned document US-B-6,623,765), or such a polyethylene glycol hydroxystearate alone (see above-mentioned document WO-A-00/78301).

By "clear composition", it is to be understood in the present Specification a composition which comprises Propofol dissolved into micelles having an average size less than 60 nm (nanometers). Preferably, such a clear composition is such that said micelles have an average size less than 30 nm and, still more preferably, less than or equal to 25 nm.

According to another embodiment of the invention, said pharmaceutical composition may comprise Propofol at a mass fraction (w/w) ranging from 0.1 % to 2.5% and, more preferably, ranging from 0.5% to 2.2%.

According to a still further embodiment of the invention, one should note that said pharmaceutical composition is devoid of any triglyceride, contrary to several above-mentioned compositions of the state of the art.

Preferably, the fatty acid of said at least one metal salt is a carboxylic monoacid having from 8 to 18 carbon atoms. More preferably, said fatty acid is a saturated or unsaturated aliphatic acid having from 16 to 18 carbon atoms and, still more preferably, said fatty acid is oleic acid.

Also preferably, the metal of said at least one metal salt is sodium or potassium.

According to a preferred embodiment of the invention, said at least one metal salt is sodium oleate.

Preferably, said at least one polyethylene glycol hydroxystearate has from 10 to 25 ethylene oxide units and, still more preferably, 15 ethylene oxide units.

Preferably, the mass ratio (w/w) surfactant system / Propofol may be less than or equal to 2.8 and, even more preferably, less than or equal to 2.4 thanks to said synergistic combination, which is even more unexpected taking into account the above-recited prior art, for obtaining such a clear composition, thus minimizing in an even more significant way said alteration phenomenon of physiological parameters, such as haemolysis and local tolerance.

More specifically, the mass ratio (w/w) of said at least one metal salt of a fatty acid (which is included in said surfactant system) to Propofol is advantageously less than or equal to 0.05 and, even more advantageously, less than or equal to 0.03.

One should note that such a reduced ratio metal salt(s) of a fatty acid / Propofol helps to still further minimize the risk of intolerance after injection.

According to another embodiment of the invention, the mass ratio (w/w) of said at least one polyethylene glycol hydroxystearate to said at least one metal salt of a fatty acid, which further characterizes said surfactant system, ranges from 10 to 130 and, preferably, from 40 to 100.

According to another embodiment of the invention, said composition further comprises a pharmaceutically acceptable adjuvant system at a mass ratio (w/w) adjuvant system / Propofol less than or equal to 2.6.

Advantageously, said adjuvant system comprises glycerin, which allows to adjust the tonicity of the composition.

Also advantageously, said adjuvant system further comprises ethanol, preferably at a mass ratio (w/w) ethanol / Propofol less than or equal to 1.

Still advantageously, said adjuvant system further comprises a polyethylene glycol of low molecular weight, preferably at a mass ratio (w/w) polyethylene glycol / Propofol less than or equal to 1.1.

Additionally, said adjuvant system may further comprises an acylated amino acid, which may preferably be a lipoaminoacid.

It should be understood that said adjuvant system may include other pharmaceutical approved ingredients designed to adjust pH, isotonicity, to protect the composition, to preserve or to control bacterial growth.

According to a still further embodiment of the invention, said pharmaceutical composition comprises water at a mass fraction (w/w) which is greater than 90% and, more advantageously, which may be greater than or equal to 93%.

One should note that this very high water content helps to make the composition of the invention safe, especially in order to reduce the risks of side effects during or after injection (pain, haemolysis, etc.).

According to another aspect of the invention, said pharmaceutical composition may also be administrated through subcutaneous or intramuscular injection.

A process according to the invention for preparing the above-defined pharmaceutical composition essentially comprises adding Propofol to an aqueous mixture comprising:
(i) water,
(ii) a surfactant system comprising at least one pharmaceutically acceptable monovalent metal salt of a fatty acid having from 5 to 23 carbon atoms and at least one polyethylene glycol hydroxystearate, and
(iii) an adjuvant system.

Advantageously, this process may comprise:
- either directly introducing said salt in this aqueous mixture,
- or reacting a suitable amount of said fatty acid with a selected amount of a sodium or potassium hydroxide solution, to obtain said salt *in situ.*

In this *in situ* embodiment of the invention, said fatty acid is first advantageously mixed with water, until a homogenous dispersion is obtained. Then sodium or potassium hydroxide are added to this dispersion and mixed therewith, until a homogenous solution containing said salt is obtained. Said polyethylene glycol hydroxystearate and said adjuvant system are subsequently mixed with this solution, until a clear homogenous solution is obtained, and Propofol is eventually added thereto and mixed until a clear homogenous solution is finally obtained.

### EXAMPLES:

### I. Preparation and properties of compositions according to the invention and of comparative or "witness" compositions:

All the following compositions were obtained thanks to traditional mixing means usually used in laboratories, and according to the following process of preparation (for 100ml of solution), unless otherwise indicated (as in example 2):
- Step (i): in a suitable vessel, one surfactant (such as PEG-15 hydroxystearate for compositions of the invention - or a poloxamer, a PEG-15 stearate or a lipoid for "witness" compositions) was introduced and mixed with a solvent or adjuvant (ethanol) until dissolution.
- Step (ii): another surfactant (such as sodium oleate for compositions of the invention - or sodium laurate for "witness" compositions), a co-solvent (for "witness" compositions) or another adjuvant (such as a PEG or glycerin) and water were added to the solution obtained in step (i) and mixed together therewith, until a clear homogenous solution was obtained.
- Step (iii): Propofol was added to the solution obtained in step (ii) and mixed until a clear homogenous solution was obtained.

The osmolarity was measured thanks to an Automatic Micro-Osmometer "ROEBLING type 13/13DR".

The micelle sizes were measured thanks to a "ZetaSizer Nano" from MALVERN.

### 1) Example 1:

A first composition according to the invention was prepared with the following formulation, in terms of mass fractions of ingredients (w/w):

| | |
|---|---|
| Active agent | |
| Propofol | 1% |
| Surfactant system | |
| PEG-15 hydroxystearate ("Solutol HS 15") | 2.25% |
| Sodium oleate | 0.05% |
| Adjuvant system | |
| Ethanol | 0.60% |
| PEG | 0.90% |
| Glycerin | 1.05% |
| Water for injection | up to 100%, i.e. 94.15% (w/w) |

This first composition contained more than 93% of water and had an osmotic value of 300 mOsm.kg⁻¹ ± 10%. Moreover, it showed clear optical properties, with an average micelle size of 23 nm. Furthermore, due to its high water content, low particle size distribution and particularly due to the very reduced amount of surfactant system (ratio surfactant system / Propofol of only 2.30) which was allowed by the inventive combination of PEG hydroxystearate and sodium oleate, this first composition appeared to be particularly safe for intravenous injections.

### 2) Example 2:

A second composition according to the invention was prepared with the following formulation in terms of mass fractions of ingredients (w/w), this second composition only differing from the first one by the use of a fatty acid not previously formed into a salt.

This second composition was prepared according to the following process of preparation:
- Step (i): in a suitable vessel, oleic acid was introduced and mixed with the water, until a homogenous dispersion was obtained.
- Step (ii): a selected amount of NaOH was added to the dispersion obtained in step (i) and mixed therewith, until a clear homogenous solution was obtained.
- Step (iii): one surfactant (PEG-15 hydroxystearate), and adjuvants (ethanol, PEG and glycerin) were added to the solution obtained in step (ii), until a clear homogenous solution was obtained.
- Step (iv): Propofol was added to the solution obtained in step (iii) and mixed until a clear homogenous solution was obtained.

| | |
|---|---|
| Active agent | |
| Propofol | 1% |
| Surfactant system | |
| PEG-15 hydroxystearate ("Solutol HS 15") | 2.25% |
| Oleic acid | 0.05% |
| Adjuvant system | |
| Ethanol | 0.60% |
| PEG | 0.90% |
| NaOH | 0.005% |
| Glycerin | 1.05% |
| Water for injection | up to 100%, i.e. 94.15% (w/w) |

This second composition contained more than 93% of water and had an osmotic value of 300 mOsm.kg⁻¹ ± 10%. Moreover, it showed clear optical properties, with an average micelle size of 27 nm.

Furthermore, due to its high water content, low particle size distribution and particularly due to the very reduced amount of surfactant system (ratio surfactant system / Propofol of only 2.30) which was allowed by the inventive combination of PEG hydroxystearate and sodium oleate, this second composition also appeared to be particularly safe for intravenous injections.

### 3) Example 3:

A third composition according to the invention was prepared with the following formulation in terms of mass fractions of ingredients (w/w), the ingredients of this composition being identical to those of the first one:

| | |
|---|---|
| Active agent | |
| Propofol | 1% |
| Surfactant system | |
| PEG-15 hydroxystearate ("Solutol HS 15") | 2.35% |
| Sodium oleate | 0.03% |
| Adjuvant system | |
| Ethanol | 0.50% |
| PEG | 1% |
| Glycerin | 1.05% |
| Water for injection | up to 100%, i.e. 94.07% (w/w) |

This third composition essentially differed from the first one in that the ratio sodium oleate / Propofol was equal to 0.03 instead of 0.05, and in that the ratio surfactant system / Propofol was equal to 2.38 instead of 2.30.

This third composition contained more than 93% of water and had an osmotic value of 300 mOsm.kg⁻¹ ± 10%. Moreover, it showed clear optical properties, with an average micelle size of 26 nm.

Further, due to its high water content, low particle size distribution and very reduced amount of surfactant system (ratio surfactant system / Propofol of only 2.38) which was allowed by the inventive combination of PEG hydroxystearate and sodium oleate, this third composition also appeared to be particularly safe for intravenous injections.

One will also note that the very low ratio sodium oleate /Propofol (0.03 here) helped to still further minimize the risk of intolerance of the injected composition.

### 4) Example 4:

A fourth composition according to the invention was prepared with the following formulation in terms of mass fractions of ingredients (w/w), the ingredients of this composition being identical to those of the third one except that the adjuvant system of this fourth composition further included a lipoaminoacid consisting of an acetylated amino acid:

| | |
|---|---|
| Active agent | |
| Propofol | 1% |
| Surfactant system | |
| PEG-15 hydroxystearate ("Solutol HS 15") | 2.35% |
| Sodium oleate | 0.025% |
| Adjuvant system | |
| Ethanol | 0.50% |
| PEG | 1% |
| Glycerin | 1.05% |
| Acylated Amino Acid | 0.025% |
| Water for injection | up to 100%, i.e. 94.05% (w/w) |

Moreover, this fourth composition essentially differed from the third one in that the ratio sodium oleate / Propofol was equal to 0.025 instead of 0.03 and in that the ratio surfactant system / Propofol was equal to 2.375 in lieu of 2.38.

This fourth composition contained more than 93% of water and had an osmotic value of 300 mOsm.kg⁻¹ ± 10%. Moreover, it showed clear optical properties, with an average micelle size of 30 nm.

Further, due to its high water content, low particle size distribution and very reduced amount of surfactant system (ratio surfactant system / Propofol of only 2.375) which was allowed by the inventive combination of PEG hydroxystearate and sodium oleate, this fourth composition also appeared to be particularly safe for intravenous injections.

Furthermore, one will note that the extremely low ratio sodium oleate / Propofol (equal to 0.025 here) helped to still further minimize the risk of intolerance of the composition after injection.

### 5) Example 5:

A fifth composition according to the invention was prepared with the following formulation in terms of mass fractions of ingredients (w/w), this composition essentially differing from the first one by the mass fraction of Propofol which was 2% instead of 1 %:

| | |
|---|---|
| Active agent | |
| Propofol | 2% |
| Surfactant system | |
| PEG-15 hydroxystearate ("Solutol HS 15") | 3.50% |
| Sodium oleate | 0.05% |
| Adjuvant system | |
| Ethanol | 1% |
| PEG | 1.50% |
| Water for injection | up to 100%, i.e. 91.95% (w/w) |

Moreover, this fifth composition essentially differed from the first one in that the ratio surfactant system / Propofol was lowered to 1.775 instead of 2.30, as well as the ratio adjuvant system / Propofol which was lowered to 1.775 in lieu of 2.55.

This fifth composition contained more than 90% of water and had an osmotic value of 300 mOsm.kg⁻¹ ± 10%. Moreover, it showed clear optical properties, with an average micelle size of 52 nm.

Furthermore, due to its high water content, low particle size distribution and extremely reduced amount of surfactant system (ratio surfactant system / Propofol of only 1.775) which was allowed by the inventive combination of PEG hydroxystearate and sodium oleate, this fifth composition also appeared to be particularly safe for intravenous injections.

### 6) Comparative example 1:

A first "witness" composition according to the known prior art was prepared with the following formulation in terms of mass fractions of ingredients (w/w), this composition only differing from said second composition of the invention in that the PEG hydroxystearate was replaced by a poloxamer "Pluronic F68/F77":

| | |
|---|---|
| Active agent | |
| Propofol | 1% |
| Surfactant system | |
| "Pluronic F68/F77" | 2.25% |
| Sodium laurate | 0.05% |
| Adjuvant system | |
| Ethanol | 0.60% |
| PEG | 0.90% |
| Glycerin | 1.05% |
| Water for injection | up to 100%, i.e. 94.15% (w/w) |

This "witness" composition contained more than 93% of water and was an opalescent solution with an average micelle size above 200 nm.

This comparative example showed the advantageous result of the synergistic combination (metal salt of a fatty acid / PEG hydroxystearate) according to said second composition of the invention, compared to the identical metal salt combined here to another nonionic surfactant (i.e. a poloxamer) which does not lead at all to a clear emulsion at such a low ratio surfactant system / Propofol of 2.30.

### 7) Comparative example 2:

A second "witness" composition according to the known prior art was prepared with the hollowing formulation in terms of mass fractions of ingredients (w/w), this composition only differing from said first composition of the invention in that PEG hydroxystearate was replaced by a PEG stearate:

| | |
|---|---|
| Active agent | |
| Propofol | 1% |
| Surfactant system | |
| PEG-15 stearate | 2.25% |
| Sodium oleate | 0.05% |
| Adjuvant system | |
| Ethanol | 0.60% |
| PEG | 0.90% |
| Glycerin | 1.05% |
| Water for injection | up to 100%, i.e. 94.15% (w/w) |

This "witness" composition contained more than 93% of water, and was an opalescent solution in which PEG-15 stearate had precipitated. The average particles size therein was above 200 nm.

This comparative example showed again the advantageous result of the synergistic combination (metal salt of a fatty acid / PEG hydroxystearate) according to said first composition of the invention, compared to the identical metal salt combined here to another nonionic surfactant (i.e. a PEG stearate) which does not lead at all to a clear emulsion at such a low ratio surfactant system / Propofol of 2.30.

### 8) Comparative example 3:

A third "witness" composition according to the known prior art was prepared with the following formulation in terms of mass fractions of ingredients (w/w), this composition only differing from said first composition of the invention in that the PEG hydroxystearate was replaced by an egg lecithin:

| | |
|---|---|
| Active agent | |
| Propofol | 1% |
| Surfactant system | |
| Lipoid "E80" | 2.25% |
| Sodium oleate | 0.05% |
| Adjuvant system | |
| Ethanol | 0.60% |
| PEG | 0.90% |
| Glycerin | 1.05% |
| Water for injection | up to 100%, i.e. 94.15% (w/w) |

This "witness" composition contained more than 93% of water and was an opalescent solution with an average micelle size above 200 nm.

This comparative example showed again the advantageous result of the synergistic combination (metal salt of a fatty acid / PEG hydroxystearate) according to said first composition of the invention, compared to the identical metal salt combined here to another nonionic surfactant (i.e. a lipoid) which does not lead at all to a clear emulsion at such a low ratio surfactant system / Propofol of 2.30.

### 9) Comparative example 4:

A fourth "witness" composition according to the known prior art was prepared with the following formulation in terms of mass fractions of ingredients (w/w), this composition essentially differing from said first composition of the invention in that sodium oleate was replaced by a poloxamer:

| | |
|---|---|
| Active agent | |
| Propofol | 1% |
| Surfactant system | |
| PEG-15 hydroxystearate ("Solutol HS 15") | 2.05% |
| Poloxamer 407 | 0.20% |
| Adjuvant system | |
| Ethanol | 0.60% |
| PEG | 0.90% |
| Glycerin | 1.05% |
| Water for injection | up to 100%, i.e. 94.10% (w/w) |

This "witness" composition contained more than 93% of water and was an opalescent solution with an average micelle size above 200 nm.

This comparative example showed again the advantageous result of the synergistic combination (metal salt of a fatty acid / PEG hydroxystearate) according to instant invention, compared to the identical PEG hydroxystearate combined here to another surfactant (i.e. a poloxamer) which does not lead at all to a clear emulsion at such a low ratio surfactant system / Propofol of 2.25.

### 10) Comparative example 5:

A fifth "witness" composition according to the known prior art was prepared with the following formulation in terms of mass fractions of ingredients (w/w), this composition only differing from that of comparative example 4 in that the mass fractions of PEG hydroxystearate and poloxamer were mutually inverted:

| | |
|---|---|
| Active agent | |
| Propofol | 1% |
| Surfactant system | |
| PEG-15 hydroxystearate ("Solutol HS 15") | 0,20% |
| Poloxamer 407 | 2.05% |
| Adjuvant system | |
| Ethanol | 0,60% |
| PEG | 0,90% |
| Glycerin | 1,05% |
| Water for injection | up to 100%, i.e. 94.10% (w/w) |

This fifth "witness" composition contained more than 93% of water and was a cloudy solution with an average micelle size above 200 nm.

This comparative example showed again the advantageous result of the synergistic combination (metal salt of a fatty acid / PEG hydroxystearate) according to instant invention, compared to the identical PEG hydroxystearate combined here to another surfactant (i.e. a poloxamer) which does not lead at all to a clear emulsion at such a low ratio surfactant system / Propofol of 2.25.

### II. Haemolysis comparative test on blood samples comprising said first composition of the invention and a marketed reference 1% Propofol composition:

### 1) Haemolysis tests conditions:

4 dogs were treated with 6 mg/kg of Propofol using said first composition of the invention, in comparison with a marketed reference composition containing 1% Propofol sold by Schering-Plough Animal Health under the trade name Rapinovet^{®} (this Rapinovet^{®} composition corresponding to the qualitative and quantitative composition of the Diprivan^{®} one for human health).

Blood samples were collected before treatment (T0) and 15 minutes after injection (T15), thus corresponding to an average concentration of approximately 1500 nanograms of Propofol per milliliter of blood.

After collecting, all the compositions were centrifuged during 10 minutes at 3200 rp.min⁻¹ and the serum samples were collected.

The titration of haemoglobin in each of the serum samples was conducted at 340 nm with an automate "LISA 200 HYCEL" calibrated with a solution of "DATATROLDIFF HYCEL".

### 2) Haemolysis results :

Table 1 hereafter contains the haemoglobin concentrations that were obtained for each of the following serum samples:
- a serum sample including the 1% Propofol composition according to example 1, and
- a serum sample including a marketed 1% Propofol composition sold by Schering-Plough Animal Health under the trade name Rapinovet^{®}.

**Table 1:**

| **Serum samples** | **Haemoglobin concentration in serum (mg.l⁻¹)** | |
|---|---|---|
| | Before treatment (T0) | 15 minutes after injection (T15) |
| | Mean of concentrations +/- standard deviation | Mean of concentrations +/- standard deviation |
| Including first composition of the invention | 2.99 +/- 0.15 | 3.36 +/- 0.48 |
| Including Rapinovet^{®} composition | 3.31 +/- 0.52 | 8.01 +/- 2.21 |

This table shows that the composition of the invention does not significantly increase the haemoglobin value and consequently does not induce any damage on the red blood cells.

Contrary to that, this table shows that the Rapinovet^{®} or Diprivan^{®} composition significantly increases haemoglobin values, which consequently indicates that they could potentially damage red blood cells.

## Claims

1. Pharmaceutical aqueous emulsion composition which comprises Propofol and a surfactant system and which is usable for administration through intravenous injection, **characterized in that** said surfactant system comprises at least one pharmaceutically acceptable monovalent metal salt of a fatty acid having from 5 to 23 carbon atoms, and at least one polyethylene glycol hydroxystearate.

2. Pharmaceutical composition according to claim 1, wherein it comprises micelles having an average size less than 60 nm.

3. Pharmaceutical composition according to claim 2, wherein said micelles have an average size less than 30 nm.

4. Pharmaceutical composition according to claim 3, wherein said micelles have an average size less than or equal to 25 nm.

5. Pharmaceutical composition according to any of the preceding claims, wherein the mass ratio (w/w) surfactant system / Propofol is less than 4.

6. Pharmaceutical composition according to claim 5, wherein the mass ratio (w/w) surfactant system / Propofol is less than or equal to 2.8.

7. Pharmaceutical composition according to claim 6, wherein the mass ratio (w/w) surfactant system / Propofol is less than or equal to 2.4.

8. Pharmaceutical composition according to any of the preceding claims, wherein the mass ratio (w/w) of said at least one metal salt of a fatty acid to Propofol is less than or equal to 0.05.

9. Pharmaceutical composition according to claim 8, wherein the mass ratio (w/w) of said at least one metal salt of a fatty acid to Propofol is less than or equal to 0.03.

10. Pharmaceutical composition according to any of the preceding claims, wherein the mass ratio (w/w) of said at least one polyethylene glycol hydroxystearate to said at least one metal salt of a fatty acid ranges from 10 to 130.

11. Pharmaceutical composition according to any of the preceding claims, wherein it further comprises a pharmaceutically acceptable adjuvant system at a mass ratio (w/w) adjuvant system / Propofol less than or equal to 2.6.

12. Pharmaceutical composition according to claim 11, wherein said adjuvant system comprises glycerin.

13. Pharmaceutical composition according to claim 11 or 12, wherein said adjuvant system further comprises ethanol at a mass ratio (w/w) ethanol / Propofol less than or equal to 1.

14. Pharmaceutical composition according to any of claims 11 to 13, wherein said adjuvant system further comprises a polyethylene glycol at a mass ratio (w/w) polyethylene glycol / Propofol less than or equal to 1.1.

15. Pharmaceutical composition according to any of claims 11 to 14, wherein said adjuvant system further comprises an acylated amino acid, such as a lipoaminoacid.

16. Pharmaceutical composition according to any of the preceding claims, wherein it comprises Propofol at a mass fraction (w/w) ranging from 0.1% to 2.5%.

17. Pharmaceutical composition according to claim 16, wherein it comprises Propofol at a mass fraction (w/w) ranging from 0.5% to 2.2%.

18. Pharmaceutical composition according to any of the preceding claims, wherein it is devoid of triglyceride.

19. Pharmaceutical composition according to any of the preceding claims, wherein the fatty acid of said at least one metal salt is a carboxylic monoacid having from 8 to 18 carbon atoms.

20. Pharmaceutical composition according to claim 19, wherein said fatty acid is a saturated or unsaturated aliphatic acid having from 16 to 18 carbon atoms, such as oleic acid.

21. Pharmaceutical composition according to any of the preceding claims, wherein the metal of said at least one metal salt is sodium or potassium.

22. Pharmaceutical composition according to claims 20 and 21, wherein said at least one metal salt is sodium oleate.

23. Pharmaceutical composition according to any of the preceding claims, wherein said polyethylene glycol hydroxystearate has from 10 to 25 ethylene oxide units.

24. Pharmaceutical composition according to claim 23, wherein said at least one polyethylene glycol hydroxystearate has 15 ethylene oxide units.

25. Pharmaceutical composition according to any of the preceding claims, wherein it comprises water at a mass fraction (w/w) greater than 90%.

26. Pharmaceutical composition according to claim 25, wherein it comprises water at a mass fraction (w/w) greater than or equal to 93%.

27. Process for preparing a pharmaceutical composition according to any of the preceding claims, **characterized in that** it comprises adding Propofol to an aqueous mixture comprising:
(i) water,
(ii) a surfactant system comprising at least one pharmaceutically acceptable monovalent metal salt of a fatty acid having from 5 to 23 carbon atoms and at least one polyethylene glycol hydroxystearate, and
(iii) an adjuvant system.

28. Process according to claim 27, wherein it comprises reacting a suitable amount of said fatty acid with a selected amount of a sodium or potassium hydroxide solution, to obtain said salt *in situ.*

## Patentansprüche

1. Pharmazeutische wässrige Emulsionszusammensetzung, die Propofol und ein Surfactant-System umfasst, und die zur Verabreichung durch intravenöse Injektion verwendbar ist, **dadurch gekennzeichnet, dass** das Surfactant-System wenigstens ein pharmazeutisch verträgliches Salz eines einwertigen Metalls und einer Fettsäure, die 5 bis 23 Kohlenstoffatome hat, und wenigstens ein Polyethylenglycolhydroxystearat umfasst.

2. Pharmazeutische Zusammensetzung nach Anspruch 1, wobei es Mizellen mit einer durchschnittlichen Größe von weniger als 60 nm umfasst.

3. Pharmazeutische Zusammensetzung nach Anspruch 2, wobei die Mizellen eine durchschnittliche Größe von weniger als 30 nm haben.

4. Pharmazeutische Zusammensetzung nach Anspruch 3, wobei die Mizellen eine durchschnittliche Größe von weniger als oder gleich 25 nm haben.

5. Pharmazeutische Zusammensetzung nach einem der vorangehenden Ansprüche, wobei das Massenverhältnis (G/G) Surfactant-System / Propofol kleiner als 4 ist.

6. Pharmazeutische Zusammensetzung nach Anspruch 5, wobei das Massenverhältnis (G/G) Surfactant-System / Propofol kleiner als oder gleich 2,8 ist.

7. Pharmazeutische Zusammensetzung nach Anspruch 6, wobei das Massenverhältnis (G/G) Surfactant-System / Propofol kleiner als oder gleich 2,4 ist.

8. Pharmazeutische Zusammensetzung nach einem der vorangehenden Ansprüche, wobei das Massenverhältnis (G/G) des wenigstens einen Metallsalzes einer Fettsäure zu Propofol kleiner als oder gleich 0,05 ist.

9. Pharmazeutische Zusammensetzung nach Anspruch 8, wobei das Massenverhältnis (G/G) des wenigstens einen Metallsalzes einer Fettsäure zu Propofol kleiner als oder gleich 0,03 ist.

10. Pharmazeutische Zusammensetzung nach einem der vorangehenden Ansprüche, wobei das Massenverhältnis (G/G) des wenigstens einen Polyethylenglycolhydroxystearats zu dem wenigstens einen Metallsalz einer Fettsäure im Bereich von 10 bis 130 liegt.

11. Pharmazeutische Zusammensetzung nach einem der vorangehenden Ansprüche, wobei sie außerdem ein pharmazeutisch verträgliches Adjuvans-System in einem Massenverhältnis (G/G) Adjuvans-System / Propofol von kleiner als oder gleich 2,6 umfasst.

12. Pharmazeutische Zusammensetzung nach Anspruch 11, wobei das Adjuvans-System Glycerin umfasst.

13. Pharmazeutische Zusammensetzung nach Anspruch 11 oder 12, wobei das Adjuvans-System außerdem Ethanol in einem Massenverhältnis (G/G) Ethanol / Propofol von kleiner als oder gleich 1 umfasst.

14. Pharmazeutische Zusammensetzung nach einem der Ansprüche 11 bis 13, wobei das Adjuvans-System außerdem ein Polyethylenglycol in einem Massenverhältnis (G/G) Polyethylenglycol /Propofol von kleiner als oder gleich 1,1 umfasst.

15. Pharmazeutische Zusammensetzung nach einem der Ansprüche 11 bis 14, wobei das Adjuvans-System außerdem eine acylierte Aminosäure, zum Beispiel eine Lipoaminosäure, umfasst.

16. Pharmazeutische Zusammensetzung nach einem der vorangehenden Ansprüche, wobei sie Propofol in einer Massenfraktion (G/G) im Bereich von 0,1% bis 2,5% umfasst.

17. Pharmazeutische Zusammensetzung nach Anspruch 16, wobei sie Propofol in einer Massenfraktion (G/G) im Bereich von 0,5% bis 2,2% umfasst.

18. Pharmazeutische Zusammensetzung nach einem der vorangehenden Ansprüche, wobei sie frei von Triglycerid ist.

19. Pharmazeutische Zusammensetzung nach einem der vorangehenden Ansprüche, wobei die Fettsäure des wenigstens einen Metallsalzes eine Monocarbonsäure ist, die 8 bis 18 Kohlenstoffatome hat.

20. Pharmazeutische Zusammensetzung nach Anspruch 19, wobei die Fettsäure eine gesättigte oder ungesättigte aliphatische Säure ist, die 16 bis 18 Kohlenstoffatome hat, zum Beispiel Ölsäure.

21. Pharmazeutische Zusammensetzung nach einem der vorangehenden Ansprüche, wobei das Metall des wenigstens einen Metallsalzes Natrium oder Kalium ist.

22. Pharmazeutische Zusammensetzung nach Anspruch 20 und 21, wobei das wenigstens eine Metallsalz Natriumoleat ist.

23. Pharmazeutische Zusammensetzung nach einem der vorangehenden Ansprüche, wobei das Polyethylenglycolhydroxystearat 10 bis 25 Ethylenoxideinheiten hat.

24. Pharmazeutische Zusammensetzung nach Anspruch 23, wobei das wenigstens eine Polyethylenglycolhydroxystearat 15 Ethylenoxideinheiten hat.

25. Pharmazeutische Zusammensetzung nach einem der vorangehenden Ansprüche, wobei sie Wasser in einer Massenfraktion (G/G) von größer als 90% umfasst.

26. Pharmazeutische Zusammensetzung nach Anspruch 25, wobei sie Wasser in einer Massenfraktion (G/G) von größer als oder gleich 93% umfasst.

27. Verfahren zur Herstellung einer pharmazeutischen Zusammensetzung nach einem der vorangehenden Ansprüche, **dadurch gekennzeichnet, dass** es Zugeben von Propofol zu einem wässrigen Gemisch, umfassend:
(i) Wasser,
(ii) ein Surfactant-System, das wenigstens ein pharmazeutisch verträgliches Salz eines einwertigen Metalls mit einer Fettsäure, die 5 bis 23 Kohlenstoffatome hat, und wenigstens ein Polyethylenglycolhydroxystearat umfasst, und
(iii) ein Adjuvans-System, umfasst.

28. Verfahren nach Anspruch 27, wobei es Umsetzen einer geeigneten Menge der Fettsäure mit einer ausgewählten Menge einer Natrium- oder Kaliumhydroxidlösung umfasst, um das Salz *in situ* zu erhalten.

## Revendications

1. Composition pharmaceutique en émulsion aqueuse qui comprend du Propofol et un système surfactant et qui peut être utilisée pour une administration par une injection intraveineuse, **caractérisée en ce que** ledit système surfactant comprend au moins un sel de métal monovalent pharmaceutiquement acceptable d'un acide gras ayant de 5 à 23 atomes de carbone, et au moins un hydroxystéarate de polyéthylène glycol.

2. Composition pharmaceutique selon la revendication 1, **caractérisée en ce qu'**elle comprend des micelles ayant une taille moyenne inférieure à 60 nm.

3. Composition pharmaceutique selon la revendication 2, **caractérisée en ce que** lesdits micelles ont une taille moyenne inférieure à 30 nm.

4. Composition pharmaceutique selon la revendication 3, **caractérisée en ce que** lesdits micelles ont une taille moyenne inférieure ou égale à 25 nm.

5. Composition pharmaceutique selon l'une quelconque des revendications précédentes, **caractérisée en ce que** le rapport en masse (p/p) système surfactant/Propofol est inférieur à 4.

6. Composition pharmaceutique selon la revendication 5, **caractérisée en ce que** le rapport en masse (p/p) système surfactant/Propofol est inférieur ou égal à 2,8.

7. Composition pharmaceutique selon la revendication 6, **caractérisée en ce que** le rapport en masse (p/p) système surfactant/Propofol est inférieur ou égal à 2,4.

8. Composition pharmaceutique selon l'une quelconque des revendications précédentes, **caractérisée en ce que** le rapport en masse (p/p) dudit au moins un sel de métal d'un acide gras au Propofol est inférieur ou égal à 0,05.

9. Composition pharmaceutique selon la revendication 8, **caractérisée en ce que** le rapport en masse (p/p) dudit au moins un sel de métal d'un acide gras au Propofol est inférieur ou égal à 0,03.

10. Composition pharmaceutique selon l'une quelconque des revendications précédentes, **caractérisée en ce que** le rapport en masse (p/p) dudit au moins un hydroxystéarate de polyéthylène glycol audit au moins un sel de métal d'un acide gras se situe dans la plage de 10 à 130.

11. Composition pharmaceutique selon l'une quelconque des revendications précédentes, **caractérisée en ce qu'**elle comprend en outre un système d'adjuvant pharmaceutiquement acceptable à un rapport en masse (p/p) du système d'adjuvant/Propofol inférieur ou égal à 2,6.

12. Composition pharmaceutique selon la revendication 11, **caractérisée en ce que** ledit système d'adjuvant comprend de la glycérine.

13. Composition pharmaceutique selon la revendication 11 ou 12, **caractérisée en ce que** ledit système d'adjuvant comprend en outre de l'éthanol à un rapport en masse (p/p) éthanol/Propofol inférieur ou égal à 1.

14. Composition pharmaceutique selon l'une quelconque des revendications 11 à 13, **caractérisée en ce que** ledit système d'adjuvant comprend en outre un polyéthylène glycol à un rapport en masse (p/p) polyéthylène glycol/Propofol inférieur ou égal à 1,1.

15. Composition pharmaceutique selon l'une quelconque des revendications 11 à 14, **caractérisée en ce que** le système d'adjuvant comprend en outre un acide aminé acylé, tel qu'un acide lipoaminé.

16. Composition pharmaceutique selon l'une quelconque des revendications précédentes, **caractérisée en ce qu'**elle comprend du Propofol à une fraction en masse (p/p) allant de 0,1 % à 2,5 %.

17. Composition pharmaceutique selon la revendication 16, **caractérisée en ce qu'**elle comprend du Propofol à une fraction en masse (p/p) allant de 0,5 % à 2,2 %.

18. Composition pharmaceutique selon l'une quelconque des revendications précédentes, **caractérisée en ce qu'**elle est dépourvue de triglycéride.

19. Composition pharmaceutique selon l'une quelconque des revendications précédentes, dans **caractérisée en ce que** l'acide gras dudit au moins un sel de métal est un acide monocarboxylique ayant de 8 à 18 atomes de carbone.

20. Composition pharmaceutique selon la revendication 19, **caractérisée en ce que** ledit acide gras est un acide aliphatique saturé ou insaturé ayant de 16 à 18 atomes de carbone, tel que l'acide oléique.

21. Composition pharmaceutique selon l'une quelconque des revendications précédentes, **caractérisée en ce que** le métal dudit au moins un sel de métal est le sodium ou le potassium.

22. Composition pharmaceutique selon les revendications 20 et 21, **caractérisée en ce que** ledit au moins un sel de métal est l'oléate de sodium.

23. Composition pharmaceutique selon l'une quelconque des revendications précédentes, **caractérisée en ce que** ledit hydroxystéarate de polyéthylène glycol a de 10 à 25 unités d'oxyde d'éthylène.

24. Composition pharmaceutique selon la revendication 23, **caractérisée en ce que** ledit au moins un hydroxystéarate de polyéthylène glycol a 15 unités d'oxyde d'éthylène.

25. Composition pharmaceutique selon l'une quelconque des revendications précédentes, **caractérisée en ce qu'**elle comprend de l'eau à une fraction en masse (p/p) supérieure à 90 %.

26. Composition pharmaceutique selon la revendication 25, **caractérisée en ce qu'**elle comprend de l'eau à une fraction en masse (p/p) supérieure ou égale à 93 %.

27. Procédé de préparation d'une composition pharmaceutique selon l'une quelconque des revendications précédentes, **caractérisé en ce qu'**il comprend l'addition de Propofol à un mélange aqueux comprenant :
(i) de l'eau,
(ii) un système surfactant comprenant au moins un sel de métal monovalent pharmaceutiquement acceptable d'un acide gras ayant de 5 à 23 atomes de carbone et au moins un hydroxystéarate de polyéthylène glycol, et
(iii) un système d'adjuvant.

28. Procédé selon la revendication 27, **caractérisé en ce qu'**il comprend la réaction d'une quantité appropriée dudit acide gras avec une quantité choisie d'une solution d'hydroxyde de sodium ou de potassium, pour obtenir ledit sel *in situ.*
